# EUROPEAN PATENT SPECIFICATION

(11) **EP 4 378 930 B1**
(45) Date of publication and mention of the grant of the patent: **10.12.2025**
(21) Application number: 22853019.2
(22) Date of filing: 01.08.2022
(51) Int. Cl.: C07D 233/64, C08G 65/22, C08G 65/332, C08G 65/333, C08L 71/02

(54) **FLUOROPOLYETHER COMPOUND**
FLUORPOLYETHERVERBINDUNG
COMPOSÉ DE FLUOROPOLYÉTHER

(30) Priority: 02.08.2021 JP 2021126704
(43) Date of publication of application: 05.06.2024
(73) Proprietor: The University of Tokyo, Bunkyo-ku, Tokyo 113-8654 (JP); DAIKIN INDUSTRIES, LTD., Osaka 530-0001 (JP)
(72) Inventor: HONDA, Satoshi, Tokyo 113-8654 (JP); IKUTA, Naoya, Tokyo 113-8654 (JP); YAMAGUCHI, Shuhei, Osaka-shi, Osaka 530-8323 (JP); HANDA, Shinya, Osaka-shi, Osaka 530-8323 (JP); YAMAUCHI, Akiyoshi, Osaka-shi, Osaka 530-8323 (JP); KISHIKAWA, Yosuke, Osaka-shi, Osaka 530-8323 (JP)
(74) Representative: Hoffmann Eitle
(86) International application number: PCT/JP2022/029540
(87) International publication number: WO 2023/013600

(56) References cited:
- WO-A1-2015/118830
- WO-A1-2020/166487
- JP-A- 2001 316 468
- JP-A- 2002 269 724
- JP-A- H0 366 641
- JP-A- S6 485 218
- KRICHELDORF HANS R.: "Cyclic polymers: Synthetic strategies and physical properties", vol. 48, no. 2, 15 January 2010 (2010-01-15), US, pages 251 - 284, XP093214971, ISSN: 0887-624X, Retrieved from the Internet <URL:https://onlinelibrary.wiley.com/doi/pdfdirect/10.1002/pola.23755> DOI: 10.1002/pola.23755
- SATOSHI HONDA ET AL: "Topology-Reset Execution: Repeatable Postcyclization Recyclization of Cyclic Polymers", ANGEWANDTE CHEMIE INTERNATIONAL EDITION, VERLAG CHEMIE, HOBOKEN, USA, vol. 58, no. 1, 29 November 2018 (2018-11-29), pages 144 - 148, XP072104677, ISSN: 1433-7851, DOI: 10.1002/ANIE.201809621
- A. E. SHAMAEV ; A. A. GLAZKOV ; A. V. IGNATENKO ; A. M. SAKHAROV: "New organosiloxanes based on 1-vinyl-2-perfluoroalkoxy-2,3,3-trifluorocyclobutanes", RUSSIAN CHEMICAL BULLETIN INTERNATIONAL EDITION SERIYA KHIMICHESKAYA, SPRINGER SCIENCE+BUSINESS MEDIA, INC, vol. 54, no. 5, 1 May 2005 (2005-05-01), pages 1250 - 1253, XP019224663, ISSN: 1573-9171, DOI: 10.1007/s11172-005-0389-y
- HONDA SATOSHI, IKUTA NAOYA, OKA MINAMI, YAMAGUCHI SHUHEI, HANDA SHINYA: "Cyclic Perfluoropolyether: Distinct Film Formability and Thermostabilization Upon Recyclable Cyclic–Linear Topological Transformation", MACROMOLECULAR RAPID COMMUNICATIONS, WILEY-VCH, DE, vol. 43, no. 2, 1 January 2022 (2022-01-01), DE , pages 2100567, XP093031778, ISSN: 1022-1336, DOI: 10.1002/marc.202100567

## Description

### Technical Field

The present disclosure relates to a novel fluoropolyether compound.

### Background Art

Compounds having a fluoropolyether group are used in a wide range of application as a fluoroelastomer, a surfacetreating agent, a lubricant, and the like, and thus fluoropolyether group-containing compounds having various structures are known (Patent Literatures 1 to 4 ).

### Citation List

### Patent Literature

Patent Literature 1: JP 2017-149839 A
Patent Literature 2: JP 2017-082194 A
Patent Literature 3: JP 2018-521183 A
Patent Literature 4: JPS6485218A

### Summary of Invention

### Technical Problem

However, known fluoropolyether group-containing compounds are chain compounds, and cyclic compounds containing a fluoropolyether group have not been known. Solution to Problem

The present invention is described in the appended claims.

### Advantageous Effect of Invention

According to the present disclosure, it is possible to provide a cyclic compound containing a fluoropolyether group.

### Brief Description of Drawing

[FIG. 1] FIG. 1 is a graph showing GPC data in examples.

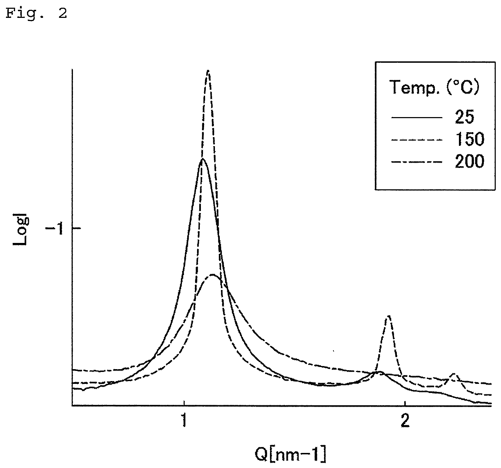

[FIG. 2] FIG. 2 is a graph showing data pertaining to emitted light measurement (temperature rise) in temperature modulation.

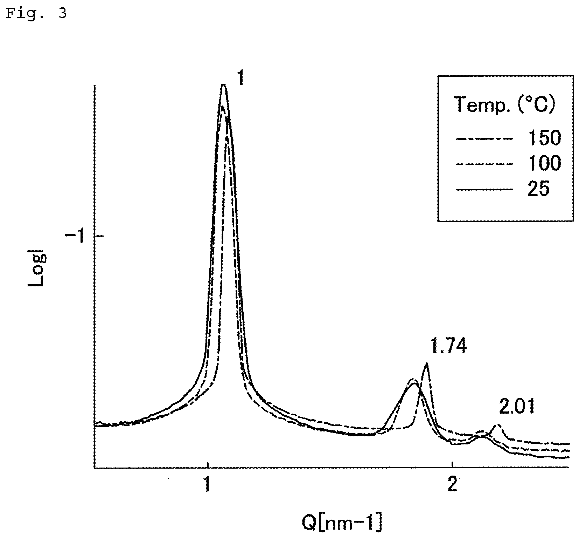

[FIG. 3] FIG. 3 is a graph showing data pertaining to emitted light measurement (temperature fall) in temperature modulation.

### Description of Embodiments

The term "monovalent organic group", as used herein, refers to a monovalent group containing carbon. The monovalent organic group may be a hydrocarbon group or a derivative thereof unless otherwise specified. A derivative of a hydrocarbon group means a group having one or more of N, O, S, Si, amide, sulfonyl, siloxane, carbonyl, carbonyloxy, and the like at the end or in the molecular chain of the hydrocarbon group. The term "organic group" refers to a monovalent organic group. The term "divalent organic group" refers to a divalent group containing carbon. Examples of the divalent organic group include, but are not limited to, a divalent group obtained by further removing one hydrogen atom from a monovalent organic group.

The term "hydrocarbon group", as used herein, refers to a group that contains carbon and hydrogen and that is obtained by removing one hydrogen atom from a hydrocarbon. The hydrocarbon group is not limited, and examples include a C₁₋₂₀ hydrocarbon group optionally substituted with one or more substituents, such as an aliphatic hydrocarbon group and an aromatic hydrocarbon group. The "aliphatic hydrocarbon group" may be either linear, branched, or cyclic, and may be either saturated or unsaturated. The hydrocarbon group may contain one or more ring structures.

As used herein, examples of the substituent of the "hydrocarbon group" include, but are not limited to, one or more groups selected from a halogen atom; and a C₁₋₆ alkyl group, a C₂₋₆ alkenyl group, a C₂₋₆ alkynyl group, a C₃₋₁₀ cycloalkyl group, a C₃₋₁₀ unsaturated cycloalkyl group, a 5 to 10-membered heterocyclyl group, a 5 to 10-membered unsaturated heterocyclyl group, a C₆₋₁₀ aryl group, and a 5 to 10-membered heteroaryl group, each of which is optionally substituted with one or more halogen atoms.

### (Fluorine-containing cyclic compound)

The present disclosure provides a fluorine-containing cyclic compound represented by the following formula (1): 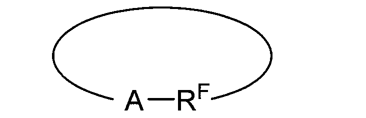 [In the formula:
A is -R² -R¹ -R² -; R¹ is a divalent organic group; R² each independently is alkylene, -O-, -COO-, - OCO-, -CONR¹¹ -, or -NR¹¹ CO-; and R¹¹ is H or a C₁₋₆ -alkyl; and
R^{F} is a divalent organic group containing a fluoropolyether group.].
A and R^{F} are bonded to each other's both ends to form a cyclic compound.

In a preferable embodiment, R¹ is -R⁴-R⁶-R⁴-. That is, the above A is a group represented by the following formula:

-R²-R⁴-R⁶-R⁴-R²-

[In the formula:
R² is each independently an alkylene group, an oxygen atom, -COO-, -OCO-, -CONR¹¹-, or -NR¹¹CO-; and
R¹¹ is a hydrogen atom or a C₁₋₆ alkyl group;
R⁴ is a single bond or a divalent organic group; and
R⁶ is -CH=CH-, -CH=CH-CH=CH-, -C≡C-, -C=C-C=-, or 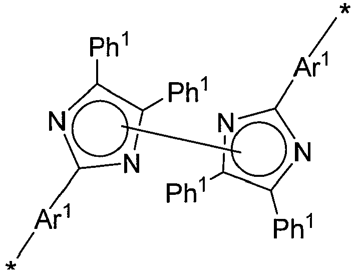 (In the formula,
Ph¹ is each independently a phenyl group optionally having a substituent;
Ar¹ is each independently a phenylene group optionally having a substituent; and
* represents a bonding site.).]

In the formula, the two imidazole rings may be bonded via any of a carbon-carbon bond, a carbon-nitrogen bond, and a nitrogen-nitrogen bond.

R² is each independently an alkylene group, an oxygen atom, -COO-, -OCO-, -CONR¹¹-, or -NR¹¹CO-.

In a preferable embodiment, R² is R^{2a} and R^{2b}. That is, A is -R^{2a}-R¹-R^{2b}-.

R^{2a} is an alkylene group, an oxygen atom, -COO-, or - CONR¹¹-, and R^{2b} is an alkylene group, an oxygen atom, -OCO-, or -NR¹¹CO-. Note that the right side of R^{2a} is bonded to R¹ and the left side of R^{2b} is bonded to R¹.

R^{2a} and R^{2b} are preferably groups corresponding to each other. That is, the combination of the above R^{2a} and R^{2b} is preferably an alkylene group and an alkylene group, an oxygen atom and an oxygen atom, -COO- and -OCO-, or -CONR¹¹- and - NR¹¹CO-.

In a preferable embodiment, R^{2a} is -CONR¹¹-, and R^{2b} is -NR¹¹CO-.

The alkylene group in R² is preferably a C₁₋₆ alkylene group and more preferably a C₁₋₃ alkylene group. The alkylene group may be linear or branched.

R¹¹ is a hydrogen atom or a C₁₋₆ alkyl group and is preferably a hydrogen atom.

The C₁₋₆ alkyl group in R¹¹ is preferably a C₁₋₃ alkyl group, more preferably a methyl group or an ethyl group, and still more preferably a methyl group. The C₁₋₆ alkyl group may be linear or branched.

R¹¹ is preferably a methyl group or a hydrogen atom and more preferably a hydrogen atom.

R⁴ is each independently a single bond or a divalent organic group.

In one embodiment, R⁴ is each independently a single bond or -(R¹⁵)ₙ₁-.

In the above formula, R¹⁵ is each independently at each occurrence a C₁₋₆ alkylene group, a 3- to 8-membered cycloalkylene group, a 3- to 8-membered cycloalkenylene group, a 3- to 8-membered heterocycloalkylene group, a 3- to 8-membered heterocycloalkenylene group, an arylene group, or a heteroarylene group; and n1 is an integer of 1 to 6.

In a preferable embodiment, R⁴ is each independently a C₁₋₆ alkylene group or -R¹⁷-R¹⁸-R¹⁹-. Note that R¹⁷ in R⁴ is bonded to R⁶.

In the above formula,
R¹⁷ is a single bond or a C₁₋₆ alkylene group;
R¹⁸ is an arylene group; and
R¹⁹ is a single bond or a C₁₋₆ alkylene group.

In one embodiment, R¹⁷ is a single bond, R¹⁸ is an arylene group, and R¹⁹ is a C₁₋₆ alkylene group.

The C₁₋₆ alkylene group in R⁴ is preferably a C₁₋₃ alkylene group and more preferably a C₁₋₂ alkylene group. The C₁₋₆ alkylene group may be linear or branched.

The C₁₋₆ alkylene group and the arylene group in R⁴ may be substituted. The substituent is not limited, and examples thereof include one or more groups selected from a halogen atom; and a C₁₋₆ alkyl group, a C₂₋₆ alkenyl group, a C₂₋₆ alkynyl group, a C₃₋₁₀ cycloalkyl group, a C₃₋₁₀ unsaturated cycloalkyl group, a 5- to 10-membered heterocyclyl group, a 5- to 10-membered unsaturated heterocyclyl group, a C₆₋₁₀ aryl group, and a 5- to 10-membered heteroaryl group, each of which is optionally substituted with one or more halogen atoms.

R⁶ is -CH=CH-, -CH=CH-CH=CH-, -C≡C-, -C=C-C=C-, or 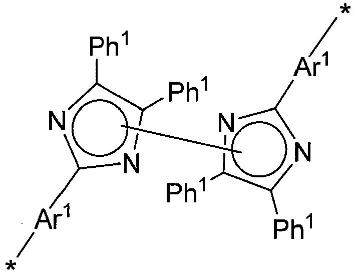 [In the formula,
Ph¹ is each independently a phenyl group optionally having a substituent;
Ar¹ is each independently a phenylene group optionally having a substituent; and
* represents a bonding site.].

In a preferable embodiment, 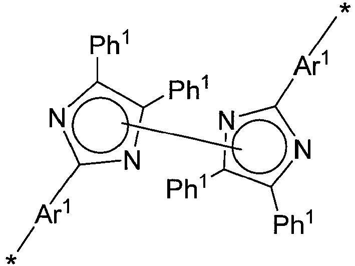 is

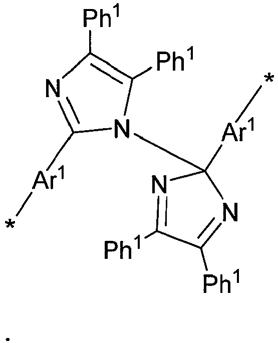

In one embodiment, Ph¹ and Ar¹ are unsubstituted.

In another embodiment, Ph¹ and Ar¹ are substituted.

The substituent in Ph¹ and Ar¹ is not limited, and examples thereof include one or more groups selected from a halogen atom; and a C₁₋₆ alkyl group, a C₂₋₆ alkenyl group, a C₂₋₆ alkynyl group, a C₃₋₁₀ cycloalkyl group, a C₃₋₁₀ unsaturated cycloalkyl group, a 5- to 10-membered heterocyclyl group, a 5- to 10-membered unsaturated heterocyclyl group, a C₆₋₁₀ aryl group, and a 5- to 10-membered heteroaryl group, each of which is optionally substituted with one or more halogen atoms.

The above R^{F} is a divalent organic group containing a fluoropolyether group.

The above fluoropolyether group is a group represented by the following formula:

- (C_{α}F_{β}R^{h}_{γ}O)_{δ}-

[In the formula:
R^{h} is a hydrogen atom, a fluorine atom, or a chlorine atom; and
α, β, γ, and δ are each independently at each occurrence any integer,
provided that at least one β is 1 or more, and
2α = β + γ is satisfied.].

The above fluoropolyether group is preferably a perfluoropolyether group. The perfluoropolyether group is a group represented by the following formula:

- (C_{α}F_{2α}O)_{δ}-

[In the formula:
α and δ are each independently at each occurrence any integer.].

In one embodiment, the above fluoropolyether group is a group represented by the following formula:

- (OC₆F₁₂)ₐ-(OC₄F₁₀)_{b}-(OC₄F₈)_{c}-(OC₃R^{h}₆)_{d}-(OC₂F₄)ₑ-(OCF₂)_{f}-

[In the formula:
R^{h} is each independently at each occurrence a hydrogen atom, a fluorine atom, or a chlorine atom; and
a, b, c, d, e, and f are each independently an integer of 0 to 200, the sum of a, b, c, d, e, and f is 1 or more, and the occurrence order of the respective repeating units enclosed in parentheses provided with a, b, c, d, e, or f is not limited in the formula.].

R^{h} is preferably a hydrogen atom or a fluorine atom and more preferably a fluorine atom. Provided that when all R^{h}s are hydrogen atoms or chlorine atoms, at least one of a, b, c, e, and f is 1 or more.

Preferably, a, b, c, d, e, and f may be each independently an integer of 0 to 100.

The sum of a, b, c, d, e, and f is preferably 5 or more, and more preferably 10 or more, and may be, for example, 15 or more or 20 or more. The sum of a, b, c, d, e, and f is preferably 200 or less, more preferably 100 or less, and even more preferably 60 or less, and may be, for example, 50 or less or 30 or less.

These repeating units may be linear or branched. For example, - (OC₆F₁₂)- may be - (OCF₂CF₂CF₂CF₂CF₂CF₂)-, - (OCF (CF₃)CF₂CF₂CF₂CF₂)-, - (OCF₂CF(CF₃)CF₂CF₂CF₂)-, - (OCF₂CF₂CF(CF₃)CF₂CF₂)-, - (OCF₂CF₂CF₂CF(CF₃)CF₂)- , - (OCF₂CF₂CF₂CF₂CF(CF₃)) -, or the like. - (OC₄F₁₀)- may be - (OCF₂CF₂CF₂CF₂CF₂)-, - (OCF (CF₃)CF₂CF₂CF₂)-, - (OCF₂CF(CF₃)CF₂CF₂)-, - (OCF₂CF₂CF(CF₃)CF₂)-, - (OCF₂CF₂CF₂CF(CF₃))-, or the like. -(OC₄F₈)- may be any of - (OCF₂CF₂CF₂CF₂)-, - (OCF(CF₃)CF₂CF₂)-, - (OCF₂CF(CF₃)CF₂)- , - (OCF₂CF₂CF(CF₃)) -, - (OC (CF₃)₂CF₂)-, - (OCF₂C(CF₃)₂)-, - (OCF (CF₃)CF(CF₃)) -, - (OCF(C₂F₅)CF₂)-, and - (OCF₂CF(C₂F₅)) -. - (OC₃F₆)- (that is, R^{h} is a fluorine atom in the above formula) may be any of - (OCF₂CF₂CF₂)-, - (OCF (CF₃)CF₂)-, and - (OCF₂CF(CF₃)) -. - (OC₂F₄)- may be any of -(OCF₂CF₂)- and - (OCF (CF₃)) - .

In a preferable embodiment, the above fluoropolyether group is each independently at each occurrence a group represented by any of the following formulae (f1) to (f5).

- (OC₃F₆)_{d}-(OC₂F₄)ₑ- (f1)
[In the formula, d is an integer of 1 to 200, and e is 0 or 1.],

   -(OC₄F₈)_{c}-(OC₃F₆)_{d}-(OC₂F₄)ₑ-(OCF₂)_{f}- (f2)
[In the formula, c and d are each independently an integer of 0 or more and 30 or less, e and f are each independently an integer of 1 or more and 200 or less,
   the sum of c, d, e, and f is 2 or more, and
   the occurrence order of the respective repeating units enclosed in parentheses provided with a subscript c, d, e, or f is not limited in the formula.],

      - (R⁶-R⁷)_{g}- (f3)
[In the formula, R⁶ is OCF₂ or OC₂F₄,
   R⁷ is a group selected from OC₂F₄, OC₃F₆, OC₄F₈, OC₅F₁₀, and OC₆F₁₂, or a combination of two or three groups independently selected from these groups, and
   g is an integer of 2 to 100.],

      - (OC₆F₁₂)ₐ-(OC₄F₁₀)_{b}-(OC₄F₈)_{c}-(OC₃F₆)_{d}-(OC₂F₄)ₑ-(OCF₂)_{f}- (f4)
[In the formula, e is an integer of 1 or more and 200 or less, a, b, c, d, and f are each independently an integer of 0 or more and 200 or less, and the occurrence order of the respective repeating units enclosed in parentheses provided with a, b, c, d, e, or f is not limited in the formula.], and

   -(OC₆F₁₂)ₐ-(OC₄F₁₀)_{b}- (OC₄F₈)_{c}-(OC₃F₆)_{d}-(OC₂F₄)ₑ-(OCF₂)_{f}- (f5)
[In the formula, f is an integer of 1 or more and 200 or less, a, b, c, d, and e are each independently an integer of 0 or more and 200 or less, and the occurrence order of the respective repeating units enclosed in parentheses provided with a, b, c, d, e, or f is not limited in the formula.].

In the above formula (f1), d is preferably 5 to 200, more preferably 10 to 100, and even more preferably 15 to 50, and is, for example, an integer of 25 to 35. In one embodiment, e is 0. In another embodiment, e is 1. The above formula (f1) is preferably a group represented by - (OCF₂CF₂CF₂)_{d}-(OCF₂CF₂)ₑ- or - (OCF(CF₃)CF₂)_{d}-(OCF(CF)₃)ₑ- and is more preferably a group represented by -(OCF₂CF₂CF₂)_{d}-(OCF₂CF₂)ₑ-.

In the formula (f2), e and f are each independently an integer of preferably 5 to 200, and more preferably 10 to 200. The sum of c, d, e, and f is preferably 5 or more, and more preferably 10 or more, and may be, for example, 15 or more or 20 or more. In one embodiment, the formula (f2) is preferably a group represented by -(OCF₂CF₂CF₂CF₂)_{c}-(OCF₂CF₂CF₂)_{d}-(OCF₂CF₂)ₑ-(OCF₂)_{f}-. In another embodiment, the formula (f2) may be a group represented by -(OC₂F₄)ₑ-(OCF₂)_{f}-.

In the formula (f3), R⁶ is preferably OC₂F₄. In the formula (f3), R⁷ is preferably a group selected from OC₂F₄, OC₃F₆, and OC₄F₈, or a combination of two or three groups independently selected from these groups, and is more preferably a group selected from OC₃F₆ and OC₄F₈. Examples of the combination of two or three groups independently selected from OC₂F₄, OC₃F₆, and OC₄F₈ include, but are not limited to, -OC₂F₄OC₃F₆-, -OC₂F₄OC₄F₈-, -OC₃F₆OC₂F₄-, - OC₃F₆OC₃F₆-, -OC₃F₆OC₄F₈-, -OC₄F₈OC₄F₈-, -OC₄F₈OC₃F₆-, - OC₄F₈OC₂F₄-, -OC₂F₄OC₂F₄OC₃F₆-, -OC₂F₄OC₂F₄OC₄F₈-, - OC₂F₄OC₃F₆OC₂F₄- -OC₂F₄OC₃F₆OC₃F₆-, -OC₂F₄OC₄F₈OC₂F₄-, - OC₃F₆OC₂F₄OC₂F₄-, -OC₃F₆OC₂F₄OC₃F₆-, -OC₃F₆OC₃F₆OC₂F₄-, and - OC₄F₈OC₂F₄OC₂F₄-. In the formula (f3), g is an integer of preferably 3 or more, and more preferably 5 or more. g is preferably an integer of 50 or less. In the formula (f3), OC₂F₄, OC₃F₆, OC₄F₈, OC₅F₁₀, and OC₆F₁₂ may be either linear or branched, and are preferably linear. In this embodiment, the formula (f3) is preferably -(OC₂F₄OC₃F₆)_{g}- or -(OC₂F₄-OC₄F₈)_{g}-.

In the formula (f4), e is an integer of preferably 1 or more and 100 or less, and more preferably 5 or more and 100 or less. The sum of a, b, c, d, e, and f is preferably 5 or more, and more preferably 10 or more, and is, for example, 10 or more and 100 or less.

In the formula (f5), f is an integer of preferably 1 or more and 100 or less, and more preferably 5 or more and 100 or less. The sum of a, b, c, d, e, and f is preferably 5 or more, and more preferably 10 or more, and is, for example, 10 or more and 100 or less.

In one embodiment, the above fluoropolyether group is a group represented by the above formula (f1).

In one embodiment, the above fluoropolyether group is a group represented by the above formula (f2).

In one embodiment, the above fluoropolyether group is a group represented by the above formula (f3).

In one embodiment, the above fluoropolyether group is a group represented by the above formula (f4).

In one embodiment, the above fluoropolyether group is a group represented by the above formula (f5).

In a preferable embodiment, R^{F} is a group represented by

-Rf¹ₚ-R^{Fa}-O_{q}-

[In the formula:
Rf¹ is a C₁₋₆ alkylene group optionally substituted with one or more fluorine atoms;
R^{Fa} is a divalent fluoropolyether group;
p is 0 or 1;
q is 0 or 1; and
m is an integer of 0 to 10.].

In the above formula, Rf¹ is a C₁₋₆ alkylene group optionally substituted with one or more fluorine atoms.

In the C₁₋₆ alkylene group optionally substituted with one or more fluorine atoms, the "C₁₋₆ alkylene group" may be linear or branched, and is preferably a linear or branched C₁₋₃ alkylene group, and more preferably a linear C₁₋₃ alkylene group.

The above Rf¹ is preferably a C₁₋₆ alkylene group substituted with one or more fluorine atoms, more preferably a C₁₋₆ perfluoroalkylene group, and still more preferably a C₁₋₃ perfluoroalkylene group.

The above C₁₋₆ perfluoroalkylene group may be linear or branched, and is preferably a linear or branched C₁₋₃ perfluoroalkylene group, more preferably a linear C₁₋₃ perfluoroalkyl group, and specifically -CF₂-, -CF₂CF₂-, or - CF₂CF₂CF₂-.

In the above formula, p is 0 or 1. In one embodiment, p is 0. In another embodiment, p is 1.

In the above formula, q is each independently 0 or 1. In one embodiment, q is 0. In another embodiment, q is 1.

In the above formula, R^{Fa} is a divalent fluoropolyether group. The divalent fluoropolyether group is the fluoropolyether group described above.

The number average molecular weight of the fluorine-containing cyclic compound represented by formula (1) of the present disclosure is not limited, and may be 300 or more, preferably 500 or more, more preferably 1,000 or more, and still more preferably 2,000 or more. In addition, the number average molecular weight of the fluorine-containing cyclic compound represented by formula (1) of the present disclosure is not limited, and may be 100,000 or less, preferably 30,000 or less, more preferably 20,000 or less, and still more preferably 10,000 or less. The number average molecular weight of the fluorine-containing cyclic compound represented by formula (1) of the present disclosure is not limited, and may be 300 to 100,000, preferably 500 to 30,000, more preferably 1,000 to 20,000, and still more preferably 2,000 to 10,000, for example. The above number average molecular weight can be measured by gel permeation chromatography (GPC).

The molecular weight distribution (weight average molecular weight/number average molecular weight) of the fluorine-containing cyclic compound represented by the above formula (1) is preferably 1.0 or more, but may be 1.1 or more or 1.2 or more. The molecular weight distribution of the fluorine-containing cyclic compound represented by the above formula (1) is preferably 4.0 or less, more preferably 3.0 or less, and still more preferably 2.0 or less. The molecular weight distribution of the fluoropolyether group-containing chain compound represented by the above formula (1) is preferably 1.0 to 4.0, more preferably 1.0 to 3.0, and still more preferably 1.0 to 2.0. The above molecular weight distribution can be measured by gel permeation chromatography (GPC).

In one embodiment, the fluorine-containing cyclic compound represented by formula (1) of the present disclosure may be crystalline. That is, the present disclosure provides a crystal of the fluorine-containing cyclic compound represented by formula (1) of the present disclosure. The crystal of the fluorine-containing cyclic compound represented by formula (1) can be confirmed through observation with a polarization microscope.

The crystal of the fluorine-containing cyclic compound represented by formula (1) can be obtained by heating the compound to a melting point thereof or higher to melt the compound and subsequently cooling the compound. For example, the crystal can be obtained by heating the compound to 200°C or to 20°C above a melting point of the compound and gradually cooling same to room temperature. The temperature decreasing rate may be preferably 20°C/minute or less and more preferably 10°C/minute or less.

The fluorine-containing cyclic compound of the present disclosure can be obtained by subjecting a fluoropolyether group-containing chain compound having a coupling functional group at both ends to coupling reaction to couple the fluoropolyether group-containing chain compound to have a cyclic structure.

Accordingly, the present disclosure provides a method for producing a fluorine-containing cyclic compound represented by the following formula (1): 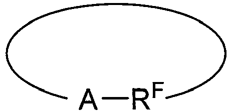 [In the formula:
A is -R² -R¹ -R² -; R¹ is a divalent organic group; R² each independently is alkylene, -O-, -COO-, - OCO-, -CONR¹¹ -, or -NR¹¹ CO-; and R¹¹ is H or a C₁₋₆ -alkyl; and
R^{F} is a divalent organic group containing a fluoropolyether group.]
the production method including subjecting a fluoropolyether group-containing chain compound having a coupling functional group at both ends to coupling reaction to couple the fluoropolyether group-containing chain compound to have a cyclic structure.

In one embodiment, the above production method is a method for producing the compound represented by formula (1).

In addition, the present disclosure provides a fluoropolyether group-containing chain compound having a coupling functional group at both ends, which can be a raw material of the fluorine-containing cyclic compound of the present disclosure.

In one embodiment, the above fluoropolyether group-containing chain compound is represented by the following formula (2):

B-R^{FA'}-(Z^{A}'-R^{FA}') _{m'}-B

[In the formula:
R^{FA'} is each independently at each occurrence a divalent organic group containing a fluoropolyether group;
m' is an integer of 0 to 10; and
B is each independently a group including a coupling functional group.].

R^{FA'} is each independently at each occurrence a divalent organic group containing a fluoropolyether group and is preferably each independently -Rf¹ₚ-R^{Fa}-O_{q}-. R^{FA'} corresponds to R^{FA} in the above formula (1). That is, R^{FA'} includes groups and aspects exemplified as R^{FA}.

The Z^{A'} corresponds to A in the above formula (1). That is, Z^{A'} includes groups and aspects exemplified as Z^{A}.

Z^{A'} is each independently at each occurrence a group represented by the following formula:

-R²-R¹-R²-

[In the formula, R¹ and R² are the same as defined above.].

In a preferable embodiment, the above Z^{A'} is each independently at each occurrence a group represented by the following formula:

-R²-R⁴-R⁶-R⁴-R²-

[In the formula, R², R⁴, and R⁶ are the same as defined above.].

B is a group represented by the following formula:

-R⁷-R⁸-R⁹

[In the formula:
R⁷ is an alkylene group, an oxygen atom, -COO-, or - CONR¹¹-;
R¹¹ is a hydrogen atom or a C₁₋₆ alkyl group;
R⁸ is a single bond or a divalent organic group;
R⁹ is a coupling functional group.].

R⁷ corresponds to R² in the above formula (1). That is, R⁷ includes groups and aspects exemplified as R².

In a preferable embodiment, R⁷ is -CONR¹¹-.

R¹¹ is a hydrogen atom or a C₁₋₆ alkyl group and is preferably a hydrogen atom.

The C₁₋₆ alkyl group in R¹¹ is preferably a C₁₋₃ alkyl group, more preferably a methyl group or an ethyl group, and still more preferably a methyl group. The C₁₋₆ alkyl group may be linear or branched.

R¹¹ is preferably a methyl group or a hydrogen atom and more preferably a hydrogen atom.

R⁸ corresponds to R⁴ and R⁵ in the above formula (1). That is, R⁸ includes groups and aspects exemplified as R⁴ and R⁵.

In one embodiment, R⁸ is each independently a single bond or -(R¹⁵)ₙ₁-.

In the above formula, R¹⁵ is each independently at each occurrence a C₁₋₆ alkylene group, a 3- to 8-membered cycloalkylene group, a 3- to 8-membered cycloalkenylene group, a 3- to 8-membered heterocycloalkylene group, a 3- to 8-membered heterocycloalkenylene group, an arylene group, or a heteroarylene group; and n1 is an integer of 1 to 6.

In a preferable embodiment, R⁸ is a C₁₋₆ alkylene group or -R¹⁷-R¹⁸-R¹⁹-. Note that R¹⁷ in R⁸ is bonded to R⁹.

In the above formula,
R¹⁷ is a single bond or a C₁₋₆ alkylene group;
R¹⁸ is an arylene group; and
R¹⁹ is a single bond or a C₁₋₆ alkylene group.

In one embodiment, R¹⁷ is a single bond, R¹⁸ is an arylene group, and R¹⁹ is a C₁₋₆ alkylene group.

The C₁₋₆ alkylene group in R⁸ is preferably a C₁₋₃ alkylene group and more preferably a C₁₋₂ alkylene group. The C₁₋₆ alkylene group may be linear or branched.

The C₁₋₆ alkylene group and the arylene group in R⁸ may be substituted. The substituent is not limited, and examples thereof include one or more groups selected from a halogen atom; and a C₁₋₆ alkyl group, a C₂₋₆ alkenyl group, a C₂₋₆ alkynyl group, a C₃₋₁₀ cycloalkyl group, a C₃₋₁₀ unsaturated cycloalkyl group, a 5- to 10-membered heterocyclyl group, a 5- to 10-membered unsaturated heterocyclyl group, a C₆₋₁₀ aryl group, and a 5- to 10-membered heteroaryl group, each of which is optionally substituted with one or more halogen atoms.

In one embodiment, R⁹ is -C=CH, -CH=CHR²⁰ (in the formula, R²⁰ is a chlorine atom, a bromine atom, an iodine atom, a hydrogen atom, or -OSO₂R²¹, preferably a chlorine atom, a bromine atom, an iodine atom, or a hydrogen atom, and more preferably a hydrogen atom; and R²¹ is a C₁₋₆ alkyl group optionally including one or more fluorine atoms or a phenyl group optionally having a substituent), -COH,
or a group represented by the following formula: 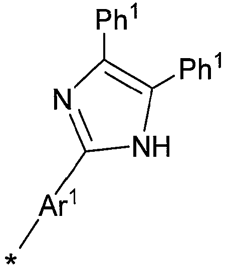 (In the formula,
Ph¹ is each independently a phenyl group optionally having a substituent;
Ar¹ is each independently a phenylene group optionally having a substituent; and
* represents a bonding site.).

In a preferable embodiment, R⁹ is a group represented by the following formula: 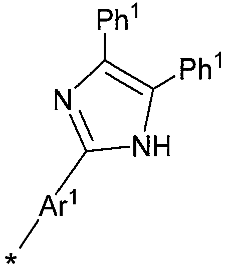 (In the formula,
Ph¹ is each independently a phenyl group optionally having a substituent;
Ar¹ is each independently a phenylene group optionally having a substituent; and
* represents a bonding site.).

In a preferable embodiment,
Z^{A'} is each independently at each occurrence -R²-R⁴-R⁶-R⁵-R³-;
R² is an alkylene group, an oxygen atom, -COO-, or - CONR¹¹-;
R³ is an alkylene group, an oxygen atom, -OCO-, or - NR¹¹CO-;
R¹¹ is a hydrogen atom or a C₁₋₆ alkyl group;
R⁴ is a single bond or a divalent organic group; and
R⁵ is a single bond or a divalent organic group;
R⁶ is a group represented by the following formula: 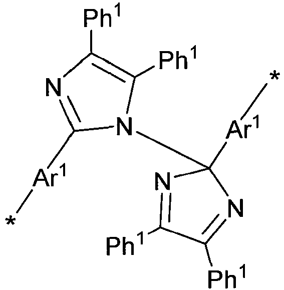 [In the formula,
   Ph¹ is each independently a phenyl group optionally having a substituent;
   Ar¹ is each independently a phenylene group optionally having a substituent; and
   * represents a bonding site.];
   B is -R⁷-R⁸-R⁹-;
   R⁷ is an alkylene group, an oxygen atom, -COO-, or - CONR¹¹-;
   R¹¹ is a hydrogen atom or a C₁₋₆ alkyl group;
   R⁸ is a divalent organic group; and
   R⁹ is a group represented by the following formula: 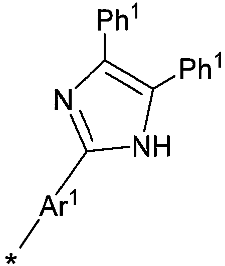 [In the formula,
      Ph¹ is each independently a phenyl group optionally having a substituent;
      Ar¹ is each independently a phenylene group optionally having a substituent; and
      * represents a bonding site.].

m' is an integer of 0 to 10, preferably an integer of 0 to 5, or an integer of 0 to 3.

In one embodiment, m' is 0.

In one embodiment, m' is 1.

In another embodiment, m' is 2 or more.

The number average molecular weight of the fluoropolyether group-containing chain compound having a coupling functional group at both ends of the present disclosure is not limited, and may be 300 or more, preferably 500 or more, more preferably 1,000 or more, and still more preferably 2,000 or more. The number average molecular weight of the fluoropolyether group-containing chain compound having a coupling functional group at both ends of the present disclosure is not limited, and may be 100,000 or less, preferably 30,000 or less, more preferably 20,000 or less, and still more preferably 10,000 or less. The number average molecular weight of the fluoropolyether group-containing chain compound having a coupling functional group at both ends of the present disclosure is not limited, and may be 300 to 100,000, preferably 500 to 30,000, more preferably 1,000 to 20,000, and still more preferably 2,000 to 10,000, for example. The above number average molecular weight can be measured by gel permeation chromatography (GPC) .

As described above, the compounds represented by formula (1) of the present disclosure can be obtained by a method including subjecting a fluoropolyether group-containing chain compound having a coupling functional group at both ends to coupling reaction to couple the fluoropolyether group-containing chain compound to have a cyclic structure. In the reaction, the fluoropolyether group-containing chain compound having a coupling functional group at both ends may form a ring through intramolecular reaction, that is, by bonding the coupling functional groups at both ends of one molecule, or may form a ring including two or more molecule units through intermolecular reaction, that is, by bonding two or more molecules to each other.

In one embodiment, the fluoropolyether group-containing chain compound having a coupling functional group at both ends is a compound represented by the above formula (2).

In one embodiment, the compounds represented by formula (1) of the present disclosure can be obtained by a method including subjecting a compound represented by the following formula:

HC≡C-R⁸-R⁷-R^{FA'}-R⁷-R⁸-C≡CH

[In the formula, R^{FA'}, R⁷, and R⁸ are the same as defined above.]
to reaction to obtain a cyclic compound including -C=C-C=C-within a ring.

In the present embodiment, R⁷ is preferably -CONR¹¹-. Note that the left side of R⁷ is bonded to R^{FA'}.

In the present embodiment, R⁸ is preferably a C₁₋₆ alkylene group.

The compound represented by formula (1) obtained by the above method is a compound in which R⁶ is -C=C-C=C-.

The above reaction can be carried out in the presence of copper bromide, copper iodide, copper chloride, or copper acetate or in the presence of a copper (I) salt under an oxygen atmosphere.

In another embodiment, the compounds represented by formula (1) of the present disclosure can be obtained by a method including subjecting a compound represented by the following formula:

CH₂=CH-R⁸-R⁷-R^{FA'}-R⁷-R⁸-CH=CH₂

[In the formula, R^{FA'}, R⁷, and R⁸ are the same as defined above.]
to reaction to obtain a cyclic compound including -HC=CH-within a ring.

In the present embodiment, R⁷ is preferably -CONR¹¹-. Note that the left side of R⁷ is bonded to R^{FA'}.

In the present embodiment, R⁸ is preferably a C₁₋₆ alkylene group.

The compounds represented by formula (1) obtained by the above method is a compound in which R⁶ is -HC=CH-.

The above reaction can be carried out using various Grubbs catalysts (first generation Grubbs catalyst, second generation Grubbs catalyst, and Hoveyda-Grubbs reagent) or a Schrock catalyst.

In another embodiment, the compound represented by formula (1) of the present disclosure can be obtained by a method including subjecting a compound represented by the following formula:

HOC-R⁸-R⁷-R^{FA}'-R⁷-R⁸-COH

[In the formula, R^{FA'}, R⁷, and R⁸ are the same as defined above.]
to reaction to obtain a cyclic compound including -HC=CH-within a ring.

In the present embodiment, R⁷ is preferably -CONR¹¹-. Note that the left side of R⁷ is bonded to R^{FA'}_{.}

In the present embodiment, R⁸ is preferably -R¹⁷-R¹8-R¹⁹-. Note that R¹⁷ in R⁸ is bonded to COH.

In the above formula,
R¹⁷ is a single bond or a C₁₋₆ alkylene group;
R¹⁸ is an arylene group; and
R¹⁹ is a single bond or a C₁₋₆ alkylene group.

In one embodiment, R¹⁷ is a single bond, R¹⁸ is an arylene group, and R¹⁹ is a C₁₋₆ alkylene group.

The C₁₋₆ alkylene group in R⁸ is preferably a C₁₋₃ alkylene group and more preferably a C₁₋₂ alkylene group. The C₁₋₆ alkylene group may be linear or branched.

The C₁₋₆ alkylene group and the arylene group in R⁸ may be substituted. The substituent is not limited, and examples thereof include one or more groups selected from a halogen atom; and a C₁₋₆ alkyl group, a C₂₋₆ alkenyl group, a C₂₋₆ alkynyl group, a C₃₋₁₀ cycloalkyl group, a C₃₋₁₀ unsaturated cycloalkyl group, a 5- to 10-membered heterocyclyl group, a 5- to 10-membered unsaturated heterocyclyl group, a C₆₋₁₀ aryl group, and a 5- to 10-membered heteroaryl group, each of which is optionally substituted with one or more halogen atoms.

The compound represented by formula (1) obtained by the above method is a compound in which R⁶ is -HC=CH-.

The above reaction can be carried out using a titanium chloride-zinc (copper) alloy.

In another embodiment, the compound represented by formula (1) of the present disclosure can be obtained by a method including subjecting a compound represented by the following formula:

R¹³-R⁸-R⁷-R^{FA'}-R⁷-R⁸-R¹³

[In the formula:
R^{FA'}, R⁷, and R⁸ are the same as defined above; and
R¹³ is a group represented by 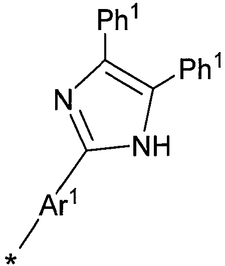 (In the formula,
   Ph¹ and Ar¹ are the same as defined above; and
   * represents a bonding site.).]

to reaction to obtain a cyclic compound including a group represented by 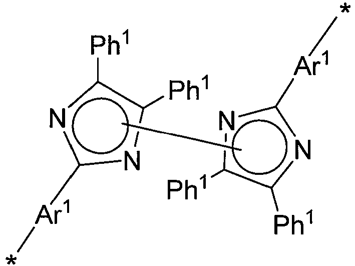 [In the formula,
   Ph¹ and Ar¹ are the same as defined above; and
   * represents a bonding site.]
within a ring.

In the present embodiment, R⁷ is preferably -CONR¹¹-. Note that the left side of R⁷ is bonded to R^{FA'}.

In the present embodiment, R⁸ is preferably a C₁₋₆ alkylene group.

Ph¹ is preferably an unsubstituted phenyl group.

Ar¹ is preferably an unsubstituted phenylene group.

The compound represented by formula (1) obtained by the above method is a compound in which R⁶ is 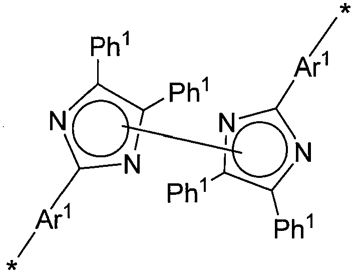 and is preferably

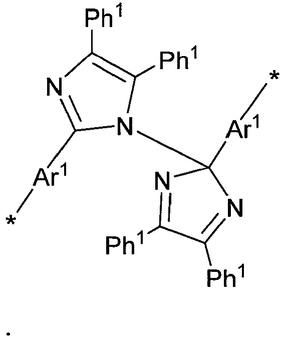

The above reaction can be carried out using a oneelectron oxidant such as potassium ferricyanide.

The lower limit of the concentration of the chain compound in the above reaction is preferably 0.1 mg/mL and may be, for example, 0.6 mg/mL or 1.0 mg/mL. The upper limit of the concentration of the chain compound is preferably 500 mg/mL, more preferably 200 mg/mL, and still more preferably 100 mg/mL, and may be, for example, 10 mg/mL or 2 mg/mL. The concentration of the chain compound in the above reaction is preferably 0.1 to 500 mg/mL, more preferably 0.1 to 200 mg/mL, and still more preferably 0.1 to 100 mg/mL, and may be, for example, 0.6 to 100 mg/mL or 0.6 to 2 mg/mL.

The present disclosure provides a composition including the fluorine-containing cyclic compound represented by formula (1) of the present disclosure.

The above composition of the present disclosure can be used, for example, for rubber materials such as a sealing agent, an adhesive, a compatibilizing agent, a waterrepelling material, a coating agent, potting gel, an O ring, packing, a gasket, a diaphragm, a valve, and a seal, but are not limited thereto.

The present disclosure provides a formed article including the fluorine-containing cyclic compound represented by formula (1) of the present disclosure or the composition of the present disclosure.

The method for forming the compound and the composition of the present disclosure is not limited, and general forming methods such as extrusion forming, injection molding, press forming, vacuum forming, and transfer forming can be used.

The shape of a formed article obtained is not limited and may be any desired shapes such as a block shape, a sheet shape, a film shape, and a bar shape and other various shapes according to application.

### Examples

The compound of the present disclosure will be described more specifically through the following Examples, but the present disclosure is not limited to these Examples. Note that, in the present Examples, the occurrence order of the repeating units (CF₂O) and (CF₂CF₂O) constituting perfluoropolyether is not limited. All the chemical formulae shown below represent average compositions.

### Example 1: Cyclization reaction of fluoropolyether group (PFPE)-containing chain compound using HABI

### 1. Lophine modification of ends of PFPE-containing chain compound, and HABI formation

A HABI-type fluoropolyether group (PFPE)-containing cyclic compound was synthesized according to the following scheme.

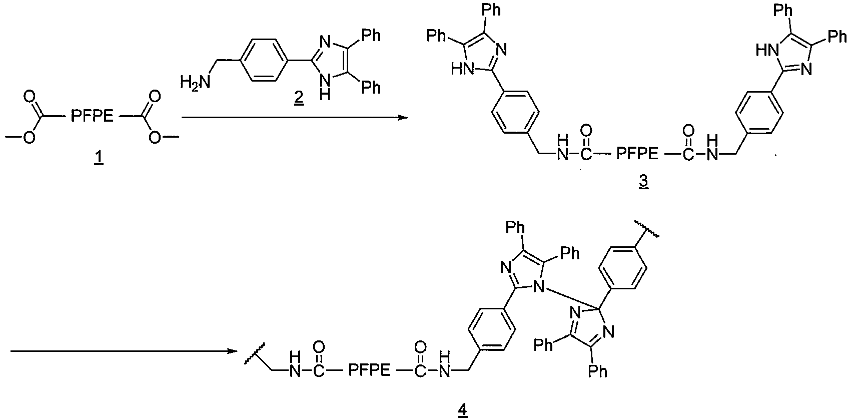

### Synthesis of triaryl imidazole

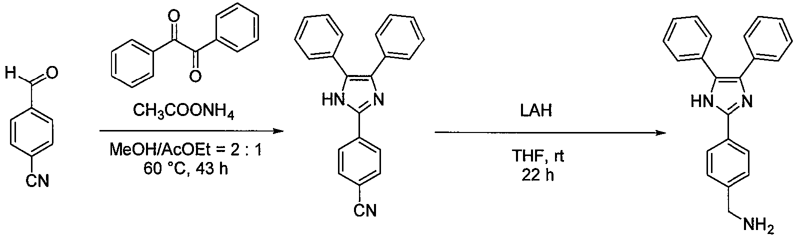

### Synthesis of 4-(4,5-diphenyl-1H-imidazole-2-yl)benzonitrile

Benzil (4.3 g, 20 mmol), 4-cyanobenzaldehyde (2.7 g, 21 mmol), and ammonium acetate (4.8 g, 62 mmol) were dissolved in a mixed solvent of methanol (60 mL) and ethyl acetate (30 mL), and heated and stirred at 60°C for two days. After returning the temperature to room temperature, filtration was carried out, and the residue was cleaned with methanol to obtain a target substance (3.6 g, 11 mmol, yield: 55%) as a white solid.
¹H NMR (500 MHz, DMSO-d6) δ 13.01 (bs, 1H), 8.25(d, J = 8.5 Hz 2H), 7.94 (d, J = 8.5 Hz 2H), 7.54-7.24 (m, 10H)

### Synthesis of 4-(4,5-diphenyl-1H-imidazole-2-yl)benzylamine

4-(4,5-Diphenyl-1H-imidazole-2-yl)benzonitrile (1.86 g, 5.79 mmol) was dissolved in ultradehydrated tetrahydrofuran (THF, 30 mL), and lithium aluminum hydroxide LAH (1.13 g, 29.8 mmol) was gradually added thereto at 0°C. The temperature was returned to room temperature, and stirring was carried out under nitrogen for 22 hours. The reaction was terminated by adding water at 0°C, followed by filtration, the residue was cleaned with ethyl acetate, and the cleaning solution was added to the filtrate, followed by cleaning with water. Next, the organic layer was dried over sodium sulfate, and sodium sulfate was removed through filtration, and the solvent was distilled off, followed by vacuum drying to obtain a target substance (1.72 g, 5.29 mmol, yield: 91%) as a white solid.
¹H NMR (500 MHz, DMSO-d6) δ 12.61 (bs, 1H), 8.01(d, J = 8.0 Hz 2H), 7.53-7.23 (m, 12H), 3.76 (s, 2H).
¹³C NMR (125 MHz, DMSO-d6) δ 145.69, 144.31, 128.41, 127.30, 125.02, 45.39
HR-MS (ESI+), calculation value as C₂₂H₁₉N₃ [M+H]+326.1652, measurement value 326.1658

### Synthesis of lophine-type fluoropolyether group (PFPE)-containing chain compound

The following compound was prepared as a PFPE compound (1) .

H₃COOC-CF₂-(OC₂F₄)ₘ-(OCF₂)ₙ-COOCH₃

(m ≈ 19, n ≈ 28)

The PFPE compound (1) (4.53 g, 1.05 mmol) was dissolved in hexafluoro-m-xylene (25 mL), and a THF solution (1.32 g, 4.06 mmol, 50 mL) of 4-(4,5-diphenyl-1H-imidazole-2-yl)benzylamine (2) was added to the solution, followed by stirring at room temperature for 20 hours under nitrogen. Thereafter, the solvent was distilled off, and the remaining solid was washed with methanol and dried to obtain a lophine-type chain PFPE compound as a white solid (4.50 g, 88%).
¹H NMR (500 MHz, Novec^{®} 7200/MeOH (10/1, v/v)) δ 10.27 (bs, 2H), 8.47 (bs, 4H), 8.02 (bs, 8H), 7.91 (bs, 4H), 7.81-7.77 (m, 12H)
¹⁹F NMR (470 MHz, (2,4-bistrifluoromethylbenzene/MeOH (10/1, v/v)) δ -54.23, -55.83, -57.55, -80.51, -82.24, -91.16, - 92.82, -128.10, -131.74.

### Synthesis of HABI-type fluoropolyether group (PFPE)-containing cyclic compound

### Synthesis of cyclization reaction HABI-type cyclic PFPE

An aqueous solution (25 mL) in which potassium hydroxide (1.29 g) and potassium ferricyanide (2.72 g) were dissolved was added to 2,4-bistrifluoromethyl benzene/methanol (v/v, 10/1) solution (66 mL) of the lophine-type chain PFPE compound (2.57 g) obtained above, followed by stirring for one hour under nitrogen, while shielding light. The organic layer was separated, and the water layer was extracted with Novec (registered trademark) 7200/methanol (10/1, v/v). The obtained organic layer was dried over magnesium sulfate and dried by distilling volatiles off under reduced pressure to obtain an initiator 1 (yellow solid, obtained amount: 0.25 g, yield: 10%).

¹H NMR (500 MHz, Novec^{®} 7200/MeOH (10/1, v/v)) δ ppm 10.22 (bs, 1H), 9.93 (bs, 1H), 8.03-7.62 (m, 26H) 7.38 (bs, 2H). ¹⁹F NMR (470 MHz, 2,4-bistrifluoromethylbenzene/MeOH (10/1, v/v)) δ ppm -54.26, -54.86, -57.58, -80.40, -82.12, -91.20, -92.86, -288.15, -131.79.

The obtained (HABI-type cyclic PFPE compound) compound (0.4 g) was dissolved in 2,4-bistrifluoromethyl benzene:methanol = 10:1, and the obtained solution was left to stand in an open system to slowly evaporate the solvent. After the sample no longer flowed, the sample was further dried under reduced pressure to prepare a film.

Synthesis of a HABI-type PFPE-containing cyclic compound (4) was confirmed by ¹H NMR and ¹⁹F NMR. Upon irradiation of the obtained yellow film-shaped solid with a blue laser (405 ± 10 nm) light, violet coloration was observed, and an absorption maximum value was observed at 570 nm through measurement with an ultraviolet-visible light spectrophotometer. The signal at g value of 2.0010 increased by irradiation with light in ESR. From this result, generation of radicals through irradiation with light was confirmed, and it was confirmed that the HABI-type PFPE-containing cyclic compound (4), a target substance, was obtained.

### 2. Confirmation of cyclization of HABI-type PFPE-containing cyclic compound

Solutions of the HABI-type PFPE-containing cyclic compound (4) prepared at different concentrations were irradiated with light (364 ± 5 nm) for 100 minutes while stirring the solutions, the solutions were stirred for 20 minutes in a dark place after the irradiation with light was stopped, and the solvent was then distilled off to obtain a yellow solid. The molecular weight distribution of the obtained solid was obtained through GPC. As a result, the smaller the concentration at the time of irradiation with light was, the smaller the polymer component became, and the polymer component became approximately constant from a concentration of 2 mg/mL. Furthermore, the ratio of the peak molecular weight (Mₚ) of the sample obtained under this concentration condition to the PFPE compound (1), which was the raw material, was calculated as 0.79, and this value was in consistency with a value derived from reduction in peak molecular weight due to general circularization. From this result, it can be said that when the concentration at the time of irradiation with light is 2 mg/mL or less, a cyclic polymer formed from one molecule of the PFPE-containing chain compound is selectively produced, and it is considered that when circularization reaction is carried out at a substrate concentration lower than this concentration, a cyclic polymer compound formed from one molecule of the PFPE-containing chain compound can be selectively produced also in general ring-closing reaction. This value is a higher value compared with the concentration of 0.5 mg/mL or less which is a general concentration when a cyclic compound is synthesized through ring-closing reaction of a chain polymer compound. This result suggests that ring-closing reaction of a single molecule using a PFPE-containing chain compound can be carried out at a high concentration, and a cyclic polymer compound can be relatively easily synthesized.

**[Table 1]**

| Molecular weight distribution measurement result from GPC | | | | | | | |
|---|---|---|---|---|---|---|---|
| Sample | Compound (4) | | | | | | Compound (1) |
| Concentratio n at time of irradiation with light (mg/mL) | 51 | 10 | 5 | 2 | 1 | 0.51 | - |
| Mn (Da) | 2,820 | 2,662 | 2,672 | 2,643 | 2,544 | 2,323 | 4,310 |
| Mw (Da) | 3,931 | 3,908 | 3,500 | 3,391 | 3,216 | 2,917 | 4,638 |
| Mz (Da) | 5,403 | 5,772 | 4,565 | 4,324 | 4,095 | 3,535 | 4,993 |
| Mp (Da) | 3,774 | 3,420 | 3,430 | 3,378 | 3,176 | 2, 998 | 4,251 |
| Mw/Mn | 1.394 | 1.468 | 1.31 | 1.283 | 1.264 | 1.256 | 1.08 |

### 3. Emitted light measurement

Emitted light measurement through temperature modulation was carried out. Specifically, the temperature was increased from 25°C to 200°C and cooled again to 25°C, and emitted light was measured at 25°C, 50°C, 100°C, 150°C, and 200°C.

A scattering pattern derived from a hexagonal phase was clearly observed at 150°C, and an isotropic phase was observed at 200°C during increasing temperature. A hexagonal scattering pattern appeared at 150°C, and scattering was broadened below 100°C during decreasing temperature.

### 4. Polarized optical microscope (POM) observation

The HABI-type PFPE-containing cyclic compound (4) was sandwiched between glass slides and heated to 200°C with a hot-stage. Thereafter, the temperature was decreased at a temperature decreasing condition of 10°C/minute. The state of the HABI-type PFPE-containing cyclic compound (4) during this period was observed with a polarized optical microscope. As a result, crystals were observed from 146.5°C.

### 5. Production of crystalline PFPE-containing compound

The HABI-type PFPE-containing cyclic compound (4) obtained in the above item 1 was heated to 200°C and melted. Thereafter, the temperature was decreased to room temperature at a temperature decreasing condition of 10°C/minute to obtain a crystalline PFPE-containing compound.

### Example 2: Glaser coupling cyclization reaction of PFPE-containing chain compound using end alkyne

### 1. End alkyne modification of PFPE

The PFPE compound (1), propargylamine (5), and metaxylene fluoride as a solvent were reacted at room temperature for three days to obtain a propargylamide-type PFPE-containing chain compound (6). Progress of reaction was confirmed through ¹⁹F NMR.

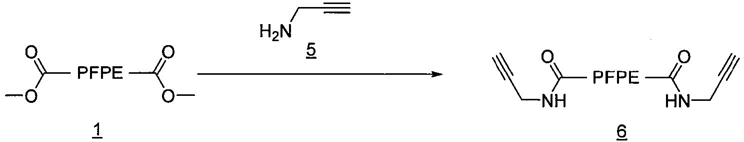

### 2. Cyclization of propargylamide-type PFPE-containing chain compound (6) (Example X)

CuBr (25 mg) and N,N,N',N",N''-pentamethyldiethylenetriamine (PMDETA) (0.04 mL) were suspended in F6-m-xylene (90 mL), followed by stirring for one hour. To this solution, a solution in which the propargylamide-type PFPE-containing chain compound (194 mg) was dissolved in F6-m-xylene (10 mL) was added. After stirring for one day, a solution obtained by suspending CuBr (65 mg) and PMDETA (0.12 mL) in F6-m-xylene (5 mL) and stirring same for one hour was added, followed by additional stirring for one day. Thereafter, a small amount of MeOH was added, cleaning with saturated saline was carried out, sodium sulfate was added to the organic layer, followed by filtration, and the filtrate was concentrated. The obtained oily product was dissolved in a mixed solvent of F6-m-xylene and acetone (about 10:1) and was passed through silica-gel, followed by concentration to obtain a milky light yellow oily product (42.3 mg). The obtained sample was subjected to ¹H NMR to confirm progress of coupling reaction.

### 3. Cyclization of propargylamide-type PFPE-containing chain compound (6) (Example Y)

The same reaction was carried out using F6-m-xylene:MeOH = 9:1. CuBr (110 mg) and PMDETA (0.15 mL) were suspended in a mixed solvent of F6-m-xylene (80 mL) and MeOH (10 mL), followed by stirring for one hour. To this solution, a solution in which the propargylamide-type PFPE-containing chain compound (190 mg) was dissolved in F6-m-xylene (10 mL) was added. After stirring for one day, a small amount of MeOH was added, cleaning with saturated saline was carried out, sodium sulfate was added to the organic layer, followed by filtration, and the filtrate was concentrated. The obtained oily product was dissolved in a mixed solvent of F6-m-xylene and acetone (about 10:1) and was passed through silica-gel, followed by concentration to obtain a milky light yellow oily product (98.3 mg). The obtained sample was subjected to ¹H NMR to confirm progress of coupling reaction.

### 4. Confirmation of cyclization

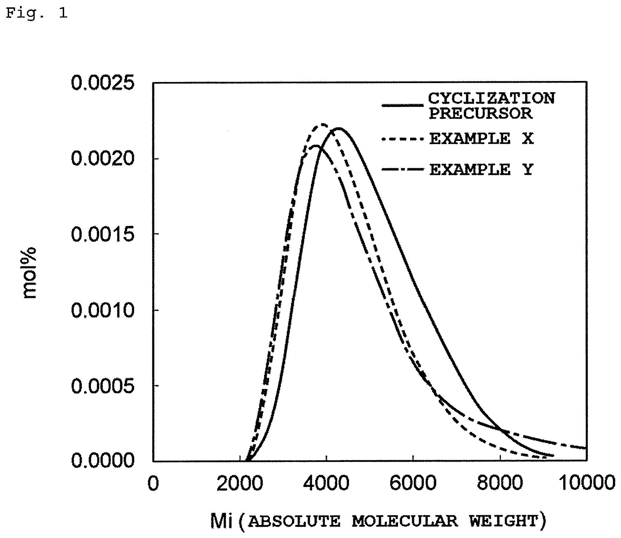

Cyclization was confirmed using GPC. From GPC data before and after reaction, the Mn, Mw, and molecular weight distribution were measured. Results are shown in the following Table 2 and FIG. 1.

**[Table 2]**

| | Cyclization precursor | Example X | Example Y |
|---|---|---|---|
| Mn (Da) | 4,663 | 4,238 | 4,279 |
| Mw (Da) | 4,969 | 4,557 | 4,722 |
| Mz (Da) | 5,295 | 4,925 | 5,300 |
| Mp (Da) | 4,665 | 4,267 | 4,102 |
| Mw/Mn | 1.066 | 1.075 | 1.104 |
| Molecular weight ratio (Mp) | - | 0.91 | 0.88 |
| >10000 | 0.0% | 0.0% | 0.8% |
| >8000 | 1.0% | 0.4% | 2.1% |
| >5000 | 33.0% | 20.5% | 19.8% |
| >3000 | 61.6% | 68.5% | 64.7% |
| >2000 | 4.4% | 10.6% | 12.7% |
| >1000 | 0.0% | 0.0% | 0.0% |
| Total | 100% | 100% | 100% |

From the above results, progress of cyclization reaction was confirmed as the proportion of low molecular weight (exceeding 2000 and 5000 or less) components increased. In addition, production of a polymerized product of the precursor compound was confirmed as the proportion of high molecular weight (exceeding 8000) components increased. These results demonstrated that cyclization progressed while including polymerized products of the precursor compound, and a composition including a cyclic compound and the polymerized products of the precursor compound was obtained.

### Industrial Applicability

The compound of the present disclosure may be used in a wide range of application such as rubber materials including a sealing agent, an adhesive, a compatibilizing agent, a water-repelling material, a coating agent, potting gel, an O ring, packing, a gasket, a diaphragm, a valve, and a seal.

## Claims

1. A compound, which is a fluorine-containing cyclic compound of formula (1): 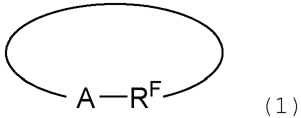 wherein
A is -R²-R¹-R²-;
R¹ is a divalent organic group;
R² each independently is alkylene, -O-, -COO-,-OCO-, -CONR¹¹-, or -NR¹¹CO-; and
R¹¹ is H or a C₁₋₆-alkyl; and
R^{F} is a divalent organic group containing a fluoropolyether group.

2. The compound of claim 1, wherein R^{F} is -Rf¹ₚ-R^{Fa}-O_{q}-;
Rf¹ is C₁₋₆-alkylene optionally substituted with F;
R^{Fa} is a divalent fluoropolyether group;
p is 0 or 1; and
q is 0 or 1.

3. The compound of claim 2, wherein R^{Fa} is a group of the formula:
-(OC₆F₁₂)ₐ-(OC₅F₁₀)_{b}-(OC₄F₈)_{c}-(OC₃R^{h}₆)_{d}-(OC₂F₄)ₑ-(OCF₂)_{f}-
wherein
R^{h} each independently is H, F or Cl;
a-f each independently are an integer of 0-200, with (a+b+c+d+e+f) ≥ 1, and the order of the repeating units in parentheses provided with a, b, c, d, e, or f is not limited.

4. The compound of claim 2 or 3, wherein R^{Fa} is each independently a group of any of formulae (f1)-(f5):
-(OC₃F₆)_{d}-(OC₂F₄)ₑ- (f1)
where d is an integer of 1-200, and e is 0 or 1,
-(OC₄F₈)_{c}-(OC₃F₆)_{d}-(OC₂F₄)ₑ-(OCF₂)_{f}- (f2)
where c and d each independently are an integer of 0-30, and e and f each independently are an integer of 1-200; (c+d+e+f) is an integer of 10-200; and the order of the repeating units in parentheses provided with a subscript c, d, e, or f is not limited,
-(R^{a}-R^{b})_{g}- (f3)
where R^{a} is OCF₂ or OC₂F₄; R^{b} is a OC₂F₄, OC₃F₆, OC₄F₈, OC₅F₁₀, OC₆F₁₂ or a combination of two or three groups selected from these groups; and g is an integer of 2-100,
-(OC₆F₁₂)ₐ-(OC₅F₁₀)_{b}-(OC₄F₈)_{c}-(OC₃F₆)_{d}-(OC₂F₄)ₑ-(OCF₂)_{f}- (f4)
where e is an integer of 1-200, a-d and f each independently are an integer of 0-200, and the order of the repeating units in parentheses provided with a, b, c, d, e, or f is not limited, and
-(OC₆F₁₂)ₐ-(OC₅F₁₀)_{b}-(OC₄F₈)_{c}-(OC₃F₆)_{d}-(OC₂F₄)ₑ-(OCF₂)_{f}- (f5)
where f is an integer of 1-200, a-e each independently are an integer of 0-200, and the order of the repeating units in parentheses provided with a, b, c, d, e, or f is not limited.

5. The compound of any of claims 1-4, wherein A is
-R²-R⁴-R⁶-R⁴-R²-;
R² each independently is alkylene, -O-, -COO-, -OCO-,-CONR¹¹-, or -NR¹¹CO-;
R¹¹ is H or C₁₋₆-alkyl;
R⁴ is each independently a single bond or a divalent organic group; and
R⁶ is -CH=CH-, -CH=CH-CH=CH-, -C≡C-, -C≡C-C≡C-, or 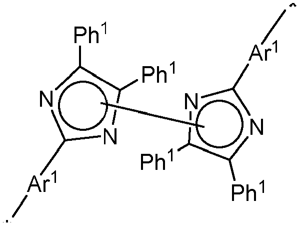
where
Ph¹ each independently is optionally substituted phenyl,
Ar¹ each independently is optionally substituted phenylene, and
* represents a bonding site.

6. The compound of any of claims 1-5, which has crystallinity.

7. The compound of any of claims 1-6, which has a molecular weight distribution of 1.0-4.0, obtained through GPC.

## Patentansprüche

1. Verbindung, welche eine fluorhaltige cyclische Verbindung der Formel (1) ist: 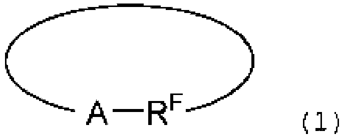 worin
A -R²-R¹-R²- ist;
R¹ eine divalente organische Gruppe ist;
R² jeweils unabhängig Alkylen, -O-, -COO-, -OCO-, -CONR¹¹- oder -NR¹¹CO- ist; und
R¹¹ H oder ein C₁₋₆-Alkyl ist; und
R^{F} eine divalente organische Gruppe ist, die eine Fluorpolyethergruppe enthält.

2. Verbindung gemäß Anspruch 1, worin R^{F} -Rf¹ₚ-R^{Fa}-O_{q}- ist; worin
Rf¹ C₁₋₆-Alkylen, optional substituiert mit F, ist;
R^{Fa} eine divalente Fluorpolyethergruppe ist;
p 0 oder 1 ist; und
q 0 oder 1 ist.

3. Verbindung gemäß Anspruch 2, worin R^{Fa} eine Gruppe der Formel:
-(OC₆F₁₂)ₐ-(OC₅F₁₀)_{b}-(OC₄F₈)_{c}-(OC₃R^{h}₆)d-(OC₂F4)ₑ- (OCF₂)_{f}-
ist, worin
R^{h} jeweils unabhängig H, F oder Cl ist;
a-f jeweils unabhängig eine ganze Zahl von 0-200 sind, wobei (a+b+c+d+e+f) ≥ 1 gilt, und die Reihenfolge der Wiederholungseinheiten in Klammern, die mit a, b, c, d, e oder f versehen sind, nicht beschränkt ist.

4. Verbindung gemäß Anspruch 2 oder 3, worin R^{Fa} jeweils unabhängig eine Gruppe gemäß irgendeiner der Formeln (f1)-(f5) ist:
-(OC₃F₆)d⁻(OC₂F₄)e⁻ (f1)
worin d eine ganze Zahl von 1-200 ist und e 0 oder 1 ist,
-(OC₄F₈)_{c}-(OC₃F₆)_{d}-(OC₂F₄)ₑ-(OCF₂)_{f}- (f2)
worin c und d jeweils unabhängig eine ganze Zahl von 0-30 sind und e und f jeweils unabhängig eine ganze Zahl von 1-200 sind; (c+d+e+f) eine ganze Zahl von 10-200 ist; und die Reihenfolge der Wiederholungseinheiten in Klammern, die mit den Indizes c, d, e oder f versehen sind, nicht beschränkt ist,
-(R^{a}-R_{b})_{g}- (f3)
worin R^{a} OCF₂ oder OC₂F₄ ist; R^{b} OC₂F₄, OC₃F₆, OC₄F₈, OC₅F₁₀, OC₆F₁₂ oder Kombinationen von zwei oder drei Gruppen, die aus diesen Gruppen ausgewählt sind, ist; und g eine ganze Zahl von 2-100 ist,
-(OC₆F₁₂)ₐ-(OC₅F₁₀)_{b}-(OC₄F₈)_{c}-(OC₃F₆)_{d}-(OC₂F₄)ₑ- (OCF₂)_{f}- (f4)
worin e eine ganze Zahl von 1-200 ist, a-d und f jeweils unabhängig eine ganze Zahl von 0-200 sind, und die Reihenfolge der Wiederholungseinheiten in Klammern, die mit a, b, c, d, e oder f versehen sind, nicht beschränkt ist, und
-(OC₆F₁₂)ₐ-(OC₅F₁₀)_{b}-(OC₄F₈)_{c}-(OC₃F₆)_{d}-(OC₂F₄)ₑ- (OCF₂)_{f}- (f5)
worin f eine ganze Zahl von 1-200 ist, a-e jeweils unabhängig eine ganze Zahl von 0-200 sind, und die Reihenfolge der Wiederholungseinheiten in Klammern, die mit a, b, c, d, e oder f versehen sind, nicht beschränkt ist.

5. Verbindung gemäß irgendeinem der Ansprüche 1-4, worin A
R²-R⁴-R⁶-R⁴-R²-
ist; worin
R² jeweils unabhängig Alkylen, -O-, -COO-, -OCO-, -CONR¹¹- oder -NR¹¹CO- ist;
R¹¹ H oder C₁₋₆-Alkyl ist;
R⁴ jeweils unabhängig eine Einfachbindung oder eine divalente organische Gruppe ist; und
R⁶ -CH=CH-, -CH=CH-CH=CH-, -C=C-, -C=C-C=C- oder 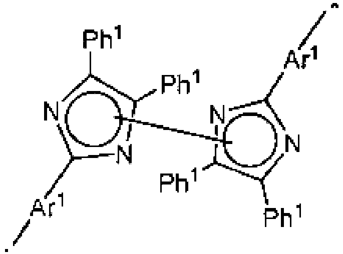
ist,
worin
Ph¹ jeweils unabhängig optional substituiertes Phenyl ist,
Ar¹ jeweils unabhängig optional substituiertes Phenylen ist und
* eine Bindungsstelle darstellt.

6. Verbindung gemäß irgendeinem der Ansprüche 1-5, welche Kristallinität aufweist.

7. Verbindung gemäß irgendeinem der Ansprüche 1-6, die eine Molekulargewichtsverteilung von 1,0-4,0 aufweist, ermittelt mittels GPC.

## Revendications

1. Composé, qui est un composé cyclique contenant du fluor de formule (1) : 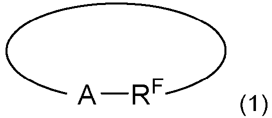 dans lequel
A est -R²-R¹-R²- ;
R¹ est un groupe organique divalent ;
R² sont chacun indépendamment alkylène, -O-, -COO-, -OCO-, -CONR¹¹-, ou -NR¹¹-CO- ; et
R¹¹ est H ou un C₁₋₆-alkyle ; et
R^{F} est un groupe organique divalent contenant un groupe fluoropolyéther.

2. Composé selon la revendication 1, dans lequel R^{F} est -Rf¹ₚ-R^{Fa}-O_{q}- ;
Rf¹ est C₁₋₆-alkylène facultativement substitué par F ;
R^{Fa} est un groupe fluoropolyéther divalent ;
p vaut 0 ou 1 ; et
q vaut 0 ou 1.

3. Composé selon la revendication 2, dans lequel R^{Fa} est un groupe de la formule :
-(OC₆F₁₂)ₐ-(OC₅F₁₀)_{b}-(OC₄F₈)_{c}-(OC₃R^{h}₆)_{d}-(OC₂F₄)ₑ-(OCF₂)_{f}-
dans lequel
R^{h} sont chacun indépendamment H, F ou CI ;
a-f sont chacun indépendamment un nombre entier de 0 à 200, avec (a+b+c+d+e+f) ≥ 1, et l'ordre des motifs de répétition entre parenthèses présentant a, b, c, d, e, ou f n'est pas limité.

4. Composé selon la revendication 2 ou revendication 3, dans lequel R^{Fa} sont chacun indépendamment un groupe de l'une quelconque des formules (f1)-(f5) :
-(OC₃F₆)_{d}-(OC₂F₄)ₑ- (f1)
où d est un nombre entier de 1 à 200, et e vaut 0 ou 1,
-(OC₄F₈)_{c}-(OC₃Fₑ)_{d}-(OC₂F₄)ₑ-(OCF₂)ᵣ- (f2)
où c et d sont chacun indépendamment un nombre entier de 0 à 30, et e et f sont chacun indépendamment un nombre entier de 1 à 200 ; et (c+d+e+f) est un nombre entier de 10 à 200 ; et l'ordre des motifs répétés entre parenthèses présentant un indice c, d, e ou f n'est pas limité,
-(R^{a}-R^{b})_{g}- (f3)
où R^{a} est OCF₂ ou OC₂F₄ ; R^{b} est un OC₂F₄, OC₃F₆, OC₄F₈, OC₅F₁₀, OC₆F₁₂ ou une combinaison de deux ou trois groupes sélectionnés parmi ces groupes ; et g est un nombre entier de 2 à 100,
-(OC₆F₁₂)ₐ-(OC₅F₁₀)_{b}-(OC₄F₈)ₑ-(OC₃F₆)_{c}-(OC₂F₄)ₑ-(OCF₂)_{f}- (f4)
où e est un nombre entier de 1 à 200, a à d et f sont chacun indépendamment un nombre entier de 0 à 200, et l'ordre des motifs de répétition entre parenthèses présentant a, b, c, d, e ou f n'est pas limité, et
-(OC₆F₁₂)ₐ-(OC₅F₁₀)_{b}-(OC₄F₈)_{c}-(OC₃F₆)_{d}-(OC₂F₄)ₑ-(OCF₂)_{f}- (f5)
où f est un nombre entier de 1 à 200, a à e sont chacun indépendamment un nombre entier de 0 à 200, et l'ordre des motifs de répétition entre parenthèses présentant a, b, c, d, e ou f n'est pas limité.

5. Composé selon l'une quelconque des revendications 1 à 4, dans lequel A est
-R²-R⁴-R⁶-R⁴-R²- ;
R² sont chacun indépendamment alkylène, -O-, -COO-, -OCO-, -CONR¹¹-, ou -NR¹¹CO- ;
R¹¹ est H ou C₁₋₆-alkyle ;
R⁴ sont chacun indépendamment une liaison unique ou un groupe organique divalent ; et
R⁶ est -CH=CH-, -CH=CH-CH=CH-, -C=C-, -C=C-C=C-, ou 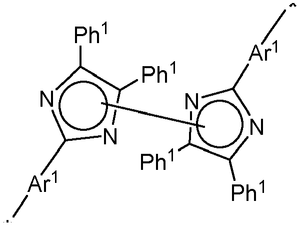
où
Ph¹ sont chacun indépendamment du phényle facultativement substitué,
Ar¹ sont chacun indépendamment du phénylène facultativement substitué, et
* représente un site de liaison.

6. Composé selon l'une quelconque des revendications 1 à 5, qui présente une cristallinité.

7. Composé selon l'une quelconque des revendications 1 à 6, qui présente une distribution de poids moléculaire de 1,0 à 4,0, obtenues par GPC.
